# EUROPEAN PATENT SPECIFICATION

(11) **EP 2 857 382 B1**
(45) Date of publication and mention of the grant of the patent: **07.03.2018**
(21) Application number: 13796662.8
(22) Date of filing: 30.05.2013
(51) Int. Cl.: C07C 69/54, C07C 67/26, C07B 61/00

(54) **HYDROXYALKYL (METH)ACRYLATE AND METHOD FOR PRODUCING SAME**
HYDROXYALKYL-(METH) ACRYLAT UND VERFAHREN ZUR HERSTELLUNG DAVON
(MÉTH)ACRYLATE D'HYDROXYALKYLE ET SON PROCÉDÉ DE FABRICATION

(30) Priority: 31.05.2012 JP 2012124981
(43) Date of publication of application: 08.04.2015
(73) Proprietor: Nippon Shokubai Co., Ltd., Osaka-shi, Osaka 541-0043 (JP)
(72) Inventor: JINNO, Hiroshi, Himeji-shi Hyogo 671-1292 (JP); ISHIDA, Tokumasa, Himeji-shi Hyogo 671-1292 (JP); TAKAKI, Hiroyuki, Himeji-shi Hyogo 671-1292 (JP); NAKAMURA, Masataka, Himeji-shi Hyogo 671-1292 (JP)
(74) Representative: Müller-Boré & Partner Patentanwälte PartG mbB
(86) International application number: PCT/JP2013/065004
(87) International publication number: WO 2013/180210

(56) References cited:
- EP-A1- 0 029 709
- WO-A1-2006/013971
- JP-A- S57 156 437
- JP-A- 2003 300 932
- JP-A- 2011 251 941
- JP-A- 2011 251 941
- US-A- 4 365 081

## Description

The present invention relates to a method for producing a highly stable hydroxyalkyl (meth)acrylate efficiently while the generation of dialkylene glycol as an impurity is inhibited.

A hydroxyalkyl (meth)acrylate such as 2-hydroxyethyl (meth)acrylate and 2-hydroxypropyl (meth)acrylate is used as a monomer for producing a poly(meth)acrylate resin. A hydroxyalkyl (meth)acrylate is characterized in having a hydroxy group among general (meth)acrylate monomers; therefore, a poly(meth)acrylate resin produced using a hydroxyalkyl (meth)acrylate as one of raw material compounds has hydroxy groups on side chains.

The above-described hydroxy group has good reactivity, since the hydroxy group is bound to a main chain through an alkylene group. Therefore, the above-described poly(meth)acrylate resin having hydroxy groups on side chains can be crosslinked, and other functional group can be introduced thereto.

For example, the above-described poly(meth)acrylate resin having hydroxy groups on side chains can be used as a component of a high performance paint, since scratch resistance and acid resistance are improved by a modification such as crosslinking. In addition, since high adhesion property and high hydrophilicity are improved by hydroxy groups, the resin can also be used as a raw material for an adhesive and contact lens and also as a processing agent for paper or fabric which contain cellulose.

In general, when a hydroxyalkyl acrylate and a hydroxyalkyl methacrylate are compared, a resin containing a hydroxyalkyl acrylate is hardened at a lower temperature and for a shorter time.

In any case, a hydroxyalkyl (meth)acrylate is a readily polymerizable compound and therefore, is problematic in that a polymerization reaction may possibly proceed during storage due to high reactivity. When a polymerization reaction once starts during storage, danger may arise since the polymerization reaction is accelerated by the reaction heat.

A hydroxyalkyl (meth)acrylate is generally produced by reacting (meth) acrylic acid with an alkylene oxide in the presence of a catalyst (Patent Document 1).

When a hydroxyalkyl (meth)acrylate is industrially produced, an impurity may be generated. Therefore, a technology to inhibit such an impurity has been developed. For example, Patent Document 2 discloses a method in which a reaction is carried out while an amount of (meth)acrylic acid relative to an amount of a catalyst is adjusted in order to inhibit the generation of dialkylene glycol mono(meth)acrylate as an impurity.

In addition, Patent Document 3 discloses a method for producing a hydroxyalkyl (meth) acrylate by using a chromium compound having a predetermined water content as a catalyst in order to reduce variability in reaction time and yield and in order to inhibit the generation of alkylene glycol as an impurity.

Patent Document 1: JP 2000-297062 A
Patent Document 2: JP 2004-10602 A
Patent Document 3: JP 2003-300932 A

JP 2011 251941 A describes a method for producing hydroxyalkyl (meth)acrylate.

US 4 365 081 A describes a process for producing 2-hydroxyalkyl acrylates or methacrylates.

EP 0 029 709 A1 describes a process for the production of hydroxy alkyl acrylates or hydroxy alkyl methacrylates.

As described above, a hydroxyalkyl (meth) acrylate is a readily polymerizable compound having high reactivity, and therefore is dangerous since a polymerization reaction may possibly proceed during storage.

Accordingly, the objective of the present invention is to provide a method for producing a highly stable hydroxyalkyl (meth)acrylate.

The present inventors studied earnestly in order to solve the above-described problem. As a result, the inventors found that when a contained amount of a dialkylene glycol which is mixed as an impurity in a hydroxyalkyl (meth)acrylate is less than the specified amount, the hydroxyalkyl (meth)acrylate has excellent stability.

In addition, the present inventors found that contamination of a dialkylene glycol remarkably deteriorates the quality of a hydroxyalkyl (meth)acrylate. Specifically, a hydroxyalkyl (meth) acrylate is used mainly as one of the monomers for producing a poly (meth) acrylate resin and the side chain hydroxy group thereof is useful for a crosslinking reaction and a functional group introducing reaction. When a hydroxyalkyl (meth)acrylate is contaminated with a dialkylene glycol, an eventually obtained poly (meth) acrylate resin is also contaminated with the dialkylene glycol. In such a case, the hydroxy group of the dialkylene glycol is reacted earlier in a polymerization reaction or a crosslinking reaction, since the reactivity of the hydroxy group of the dialkylene glycol is higher than that of the hydroxy group of the hydroxyalkyl (meth)acrylate. As a result, a poly(meth)acrylate resin with desired characteristics can not be obtained.

Furthermore, various methods have been developed as a method for producing a hydroxyalkyl (meth)acrylate while repressing an impurity, but there is conventionally no technology in which a dialkylene glycol generated as a by-product from an alkylene oxide is recognized as an impurity and by which such a dialkylene glycol is reduced.

Accordingly, the objective of the present invention is to provide a method for producing a hydroxyalkyl (meth)acrylate efficiently while the generation of a dialkylene glycol as an impurity is inhibited.

In order to solve the above-described problem, the present inventors completed the present invention by finding that the cause of the generation of a dialkylene glycol as a by-product in the production of a hydroxyalkyl (meth)acrylate is water which is slightly contained in raw material compounds, and that the generation of a dialkylene glycol can be remarkably inhibited by using raw material compounds of which water content is reduced to the prescribed amount.

Hereinafter, the present invention is described.

[1] A method for producing a hydroxyalkyl (meth)acrylate,
   comprising the step of reacting (meth)acrylic acid with an alkylene oxide in the presence of a catalyst,
   wherein a water content in a whole reaction mixture is adjusted to be not more than 0.05 mass%.
[2] The method according to the above [1], an initial charged amount of the (meth) acrylic acid is adjusted to be not more than 90 mass% of the whole use amount, and after all of or a part of the alkylene oxide is added, the rest (meth)acrylic acid is added.
[3] The method according to the above [1] or [2], after the (meth)acrylic acid is reacted with the alkylene oxide, the hydroxyalkyl (meth) acrylate is separated as a target compound from the reaction mixture, and then the residual reaction mixture is used in the next reaction.

The hydroxyalkyl (meth)acrylate produced in the present invention is superior in stability and safer, since the progress of a polymerization reaction is inhibited, for example, when the hydroxyalkyl (meth)acrylate is stored.

In addition, according to the present invention method, a hydroxyalkyl (meth) acrylate can be effectively produced while the generation of a dialkylene glycol as an impurity is inhibited by selecting an appropriate raw material compound or preliminarily treating a raw material compound. For example, a hydroxyalkyl (meth)acrylate of which contained amount of a dialkylene glycol is reduced is suitable as a raw material compound of a poly (meth) acrylate resin, since the hydroxy group of a dialkylene glycol is higher in reactivity than that of a hydroxyalkyl (meth)acrylate and therefore is the cause of a side reaction.

Accordingly, the present invention is industrially excellent as a technology which relates to the production of a high quality hydroxyalkyl (meth)acrylate.

The hydroxyalkyl (meth)acrylate produced in the present invention is characterized in that the contained amount of a dialkylene glycol which is an impurity is 0.05% by mass or less.

A hydroxyalkyl (meth)acrylate is a readily polymerizable compound and dangerous, since a polymerization reaction may possibly start during storage. The present inventors found that the causative substance which deteriorates the stability of a hydroxyalkyl (meth)acrylate is a dialkylene glycol mixed as an impurity, and that an undesirable polymerization reaction of a hydroxyalkyl (meth)acrylate can be inhibited and the stability thereof can be improved by reducing the content of such a dialkylene glycol, though the reason for the findings is not necessarily clear.

As described above, since the hydroxy group of a dialkylene glycol is more reactive than the side chain hydroxy group of a hydroxyalkyl (meth)acrylate, there is a problem that a desired polymer cannot be obtained when a hydroxyalkyl (meth) acrylate which is contaminated with a dialkylene glycol is subjected to a polymerization reaction. Such a side reaction can be inhibited by the present invention, since the contained amount of a dialkylene glycol in the hydroxyalkyl (meth) acrylate produced in the present invention is reduced.

Furthermore, when a hydroxyalkyl (meth)acrylate which is contaminated with a dialkylene glycol is subjected to a polymerization reaction as one of monomers, the dialkylene glycol causes a dehydration-condensation reaction, a transesterification reaction or the like with a raw material compound or a polymerization reaction product. As a result, there is a problem that the properties of the target polymer are adversely affected due to the change in the polymer's structure. Such a problem can be also solved by using the hydroxyalkyl (meth)acrylate produced in the present invention, since the contained amount of a dialkylene glycol is reduced.

In the present invention, the term "(meth)acrylate" means at least one of acrylate and methacrylate, namely acrylate, methacrylate, or a mixture of acrylate and methacrylate.

The contained amount of a dialkylene glycol in the hydroxyalkyl methacrylate produced in the present invention is preferably not more than 0.02% by mass, more preferably not more than 0.015% by mass, and even more preferably not more than 0.010% by mass. The contained amount of a dialkylene glycol in the hydroxyalkyl acrylate produced in the present invention is preferably not more than 0.05% by mass, and more preferably not more than 0.03% by mass.

When the contained amount of a dialkylene glycol in the hydroxyalkyl (meth)acrylate produced in the present invention exceeds 0.05% by mass, the stability of the hydroxyalkyl (meth)acrylate becomes insufficient and danger may arise, since a polymerization reaction may possibly proceed during storage. In addition, it is not preferable that the contained amount exceeds 0.05% by mass, since the hydroxy group of a dialkylene glycol reacts first or causes a dehydrative polymerization reaction with a raw material monomer or a polymer during polymerization reaction or crosslinking reaction; as a result, a poly(meth)acrylate resin having desired characteristics can not be obtained.

On the other hand, the lower limit of the contained amount of a dialkylene glycol in the hydroxyalkyl (meth)acrylate produced in the present invention is not particularly limited and is ideally 0% by mass. For example, the lower limit may be determined to be a detection limit. Although a detection limit of a dialkylene glycol varies depending on a measuring instrument and the like, for example, a detection limit of a general gas chromatography is 0.0001% by mass. The contained amount may be not less than 0.0005% by mass or not less than 0.001% by mass.

Needless to say, a higher purity of the hydroxyalkyl (meth)acrylate produced in the present invention is preferred. Specifically, the purity of the hydroxyalkyl (meth)acrylate produced in the present invention is preferably not less than 95.0% by mass, more preferably not less than 96.5% by mass, and even more preferably not less than 97.5% by mass, not less than 98.5% by mass or not less than 99.5% by mass.

The hydroxyalkyl (meth)acrylate produced in the present invention can be produced by sufficiently purifying to reduce the contained amount of a dialkylene glycol to the prescribed value. However, in particular, it is not suitable for industrial mass production of a hydroxyalkyl (meth)acrylate, since yield would be reduced. Therefore, it is preferred that the hydroxyalkyl (meth)acrylate produced in the present invention is produced by a method which is capable of producing the hydroxyalkyl (meth)acrylate efficiently while the generation of a dialkylene glycol is repressed. As such a method, the present invention method is used.

The method according to the present invention for producing a hydroxyalkyl (meth)acrylate comprises the step of reacting (meth)acrylic acid with an alkylene oxide in the presence of a catalyst. Hereinafter, the present invention method is described step by step in order.

The catalyst to be used in the production method of the present invention is not particularly restricted, and is exemplified by a catalyst which contains at least one compound selected from the group consisting of a chromium compound, an iron compound, a yttrium compound, a lanthanum compound, a cerium compound, a tungsten compound, a zirconium compound, a titanium compound, a vanadium compound, a phosphorus compound, an aluminum compound, a molybdenum compound and an amine compound.

The compound which can be used for the catalyst is exemplified by a powder; a halogen ion salt such as a chloride ion salt; an organic acid salt such as a formate, an acetate and a (meth) acrylate; an inorganic acid salt such as a nitrate and a sulfate; a coordination compound having a ligand such as acetylacetone; and an alkoxide such as a propoxide and a butoxide; of the above-described metal. The above phosphorus compound is exemplified by an alkylphosphine such as trimethylphosphine and triphenylphosphine, and a quaternary phosphonium salt such as a (meth)acrylic acid salt of the alkylphosphine.

When the above amine compound is used in addition to the catalyst which contains a metal, a synergistic effect on catalytic activity is demonstrated. Specifically, a reaction conversion ratio is increased and a reaction selectivity is improved. Such an amine compound is not restricted as long as the compound has an amine functional group in the molecule, and exemplified by a trialkylamine compound; a cyclic amine compound such as pyridine; a quaternary ammonium salt thereof; and a basic anion exchange resin which contains at least one basic functional group such as a tertiary amino group, a quaternary ammonium group and a pyridinium group.

A catalyst is classified into a homogeneous catalyst, which dissolve in a reaction mixture, and a heterogeneous catalyst, at least a part of which remains undissolved. In the present invention, it is preferred to use a homogeneous catalyst since the reaction advances more efficiently.

The use amount of the catalyst relative to (meth) acrylic acid is not particularly limited and may be adjusted appropriately. For example, in the case of a homogeneous catalyst, the use amount thereof relative to the whole use amount of (meth)acrylic acid is preferably not less than 0. 001 mol% and not more than 10 mol%. When the use amount is less than 0.001 mol%, the productivity may be possibly decreased since a reaction rate may become too slow and a reaction time may become longer. On the other hand, when the use amount exceeds 10 mol%, a reaction selectivity of a by-product may be possibly increased. The use amount is more preferably not less than 0.005 mol%, even more preferably not less than 0.01 mol%, and more preferably not more than 5 mol%, even more preferably not more than 3 mol%. In the case of a heterogeneous catalyst, the use amount relative to the whole use amount of (meth) acrylic acid is preferably not less than 5% by mass and preferably not more than 80% by mass, and more preferably not less than 10% by mass and more preferably not more than 70% by mass, for the same reason as that described for a homogeneous catalyst.

In the production method of the present invention, a polymerization inhibitor is preferably used. The polymerization inhibitor is not particularly restricted, and any one which is commonly used for the production of (meth) acrylic acid or derivative thereof may be used. For example, the polymerization inhibitor to be used is exemplified by a phenol compound such as hydroquinone and hydroquinone monomethyl ether; a paraphenylenediamine such as N-isopropyl-N'-phenyl-p-phenylenediamine; a phenylamine compound such as thiodiphenylamine and phenothiazine; a copper dialkyldithiocarbamate such as copper dibutyldithiocarbamate, copper diethyldithiocarbamate and copper dimethyldithiocarbamate; a N-oxyl compound such as 2,2,4,4-tetramethylazetidine-1-oxyl. In addition, a gas which contains molecular oxygen may also be used. As such a gas, air, oxygen itself, a mixed gas of oxygen and an inert gas, or the like may be used. In the case of using a gas containing molecular oxygen, the gas may be preferably bubbled into the reaction mixture. One of the polymerization inhibitors may be used individually, and alternatively two or more may be used in combination.

The use amount of a polymerization inhibitor may be adjusted appropriately. For example, the use amount relative to the whole use amount of (meth) acrylic acid is preferably not less than 0.0001% by mass and not more than 1% by mass, and more preferably not less than 0.001% by mass and not more than 0.5% by mass.

In the present invention method, (meth) acrylic acid is reacted with an alkylene oxide. (Meth) acrylic acid serves also as a solvent during the reaction, since (meth)acrylic acid has a relatively low melting point.

The term "alkylene oxide" means ethylene oxide or a compound in which a hydrogen atom on the methylene group of ethylene oxide is substituted with an alkyl group. The number of carbon atom of the alkylene oxide is preferably not less than 2 and not more than 6, more preferably not less than 2 and not more than 4, and particularly preferably 2 or 3. In other words, ethylene oxide or propylene oxide is particularly preferred as the alkylene oxide. 2-Hydroxyethyl (meth)acrylate and 2-hydroxypropyl (meth)acrylate produced therefrom have particularly high usefulness as a raw material compound for a poly(meth)acrylate resin, which is a component of a paint or an adhesive.

The use amounts of (meth)acrylic acid and an alkylene oxide may be adjusted appropriately. For example, the use amount of an alkylene oxide per mol of (meth) acrylic acid is preferably not less than 1.0 mol and not more than 10 mol. When the use amount is less than 1.0 mol, the reaction between (meth)acrylic acid and an alkylene oxide may possibly become difficult to proceed. On the other hand, when the use amount exceeds 10 mol, economic disadvantages may possibly be caused due to necessity of an alkylene oxide recovery step or the like. The use amount is preferably not more than 5.0 mol, more preferably not more than 3.0 mol, and particularly preferably not more than 2.0 mol.

A solvent may be used in the present invention method. Such a solvent may be chosen appropriately and is not particularly restricted. For example, an aliphatic hydrocarbon such as hexane; an aromatic hydrocarbon such as benzene and toluene may be used.

In the present invention, the water content in the whole reaction mixture in the above-described step, namely, the step of reacting (meth) acrylic acid with an alkylene oxide in the presence of a catalyst, is adjusted to be not more than the prescribed concentration by using a raw material compound with a controlled water content or using a dried raw material compound. More specifically, the water content in the whole reaction mixture is adjusted to be not more than 0.05% by mass. When a hydroxyalkyl methacrylate is produced, it is preferred to adjust the water content in the whole reaction mixture to not more than 0.025% by mass, more preferably not more than 0.020% by mass, and even more preferably not more than 0.015% by mass. When a hydroxyalkyl acrylate is produced, it is preferred to adjust the water content in the whole reaction mixture to not more than 0.04% by mass, and more preferably not more than 0.035% by mass. According to the present inventors' findings, slight amount of water which exists in the reaction mixture causes the generation of a dialkylene glycol as an impurity. Therefore, it is necessary to reduce the water content to not more than the prescribed value. In the present invention, not only (meth) acrylic acid and an alkylene oxide but all the compounds contained in the reaction mixture, such as a catalyst, may be referred to as raw material compounds in some cases.

On the other hand, the lower limit of the water content is not particularly limited and is ideally 0% by mass. For example, the lower limit may be determined to be a detection limit. Although a detection limit of water content varies depending on a measuring instrument and the like, for example, a detection limit of a general Karl Fischer moisture meter is 0.0001% by mass. The water content may be not less than 0.0001% by mass or not less than 0.001% by mass.

The method for measuring the water content of each raw material compound is not particularly restricted, and an ordinary method may be used. For example, a Karl Fischer method using a Karl Fischer moisture meter may be employed. The water content in the whole reaction mixture may be measured with respect to a reaction mixture just before the initial stage of the reaction or at the initial stage of the reaction. Alternatively, the water content of each raw material compound may be measured to calculate the water content in the whole reaction mixture from the measured values.

When the water content in the whole reaction mixture exceeds the prescribed value, the water content is preferably decreased by drying a raw material compound. The raw material compound to be dried is preferably a catalyst and a polymerization inhibitor, and particularly a catalyst, since there is less possibility of thermal decomposition and a side reaction. When the water content in (meth)acrylic acid is high, it is preferred to dehydrate the (meth)acrylic acid by a method other than heating or to use (meth)acrylic acid with a low water content.

The method for drying a raw material compound is not particularly restricted, and an ordinary method may be used. For example, each raw material compound may be heated at a temperature which is not higher than the boiling point of the compound. During such heating, pressure may be reduced. It is preferred to preliminarily dry a raw material compound of which water content is particularly high or which can be easily dried, rather than to dry the reaction mixture. Such a raw material compound to be dried may be selected from those of which solid state is maintained under normal heating condition due to the relatively high boiling point thereof or of which water content is relatively high. The above raw material compound having relatively high boiling point is exemplified by a catalyst, and the above raw material compound having relatively high water content may be selected from a polymerization inhibitor. The drying means is not particularly restricted, and may be chosen appropriately. For example, a dryer may be used. When the water content in an alkylene oxide is high, it is particularly preferred to dehydrate the alkylene oxide by other method than heating with high temperature, since an alkylene oxide generally has low boiling point and therefore it is difficult to be dried by heating.

A dryer is not particularly restricted, and is exemplified by a box type dryer and a spray type dryer. When a raw material compound contains an organic substance, an explosion-proof type dryer is preferably used to carry out drying, or drying is preferably carried out while the concentration of the organic compound gas in a gas phase portion of dryer is kept out of an explosion range under a flow of an inert gas or the like. A drying condition may be determined appropriately depending on a raw material compound to be dried, and is not particularly restricted. For example, it is preferred that temperature is not less than 70 °C and not more than 110°C, and time is not shorter than 30 minutes and not longer than 1 hour. The drying temperature is more preferably not less than 90°C and not more than 110°C.

In the present invention method, (meth) acrylic acid is reacted with an alkylene oxide in the presence of a catalyst. The whole use amount of the raw material compounds may be used to be reacted from the initial stage of the reaction, or alternatively, only a part of the compounds may be used at the initial stage of the reaction.

For example, in general, the whole use amount of a homogeneous catalyst is preliminarily charged into a reactor before the initial stage of the reaction. However, the initial charged amount may be a part of the whole use amount as long as catalytic activity can be exerted, for example, when a desirable ratio relative to (meth)acrylic acid can be satisfied. In such a case, the rest catalyst may be added during the reaction proceedings. A homogeneous catalyst may be preliminarily dissolved in (meth) acrylic acid or an alkylene oxide to be supplied into a reactor. For example, a homogeneous catalyst may be dissolved in (meth) acrylic acid as a raw material compound in a dissolving vessel other than a reactor, and then added into the reactor together with the (meth)acrylic acid.

With respect to a heterogeneous catalyst, similarly, the whole use amount thereof may be used from the initial stage of the reaction, or only a part of the whole use amount may be charged into a reactor at the initial stage of the reaction and the rest catalyst may be added little by little.

A polymerization inhibitor is used for preventing polymerization of (meth)acrylic acid or a hydroxyalkyl (meth) acrylate as the target compound to be produced. Therefore, the whole use amount of a polymerization inhibitor may be added into a reactor before the initial stage of the reaction, or alternatively, a polymerization inhibitor may be added little by little according to supply of (meth)acrylic acid or the like.

The whole use amount of (meth)acrylic acid may be used from the initial stage of the reaction. However, it is preferred to supply a part of the whole use amount into a reactor at the initial stage of the reaction, and after the initial stage of the reaction, the rest (meth)acrylic acid is added little by little. According to the findings of the present inventors, the generation of a dialkylene glycol as an impurity can be inhibited in the case of using (meth)acrylic acid little by little more efficiently than the case of using the whole use amount thereof from the initial stage of the reaction.

For example, it is preferred that an initial charged amount of the (meth) acrylic acid is adjusted to be not more than 90 mass% of the whole use amount, and all of or a part of an alkylene oxide is added to start the reaction and then, the rest (meth)acrylic acid is added. More specifically, it is preferred that not more than 90 mass% of the whole use amount is added at the initial stage of the reaction to be once reacted with an alkylene oxide, and then the rest (meth) acrylic acid may be added all at once or in not less than two portions. The initial charged amount is more preferably not more than 50% by mass, even more preferably not more than 40% by mass, and particularly preferably not more than 35% by mass. The lower limit of the initial amount is not particularly limited, and is preferably not less than 1% by mass since when the use amount is too small, the number of addition is increased so that the productivity may be possibly lowered. The initial amount is more preferably not less than 2% by mass, and particularly preferably not less than 5% by mass. The addition of a raw material compound in two or more portions in such a manner is referred to as successive addition. (Meth)acrylic acid may be added continuously at a constant rate or non-constant rate. Alternatively, a part of (meth) acrylic acid may be added at once, and the rest (meth) acrylic acid is added continuously. In the present invention, an initial charged amount means an amount of a raw material compound which exists in a reactor at the initial stage of the reaction described later. When (meth)acrylic acid is added continuously at least at the initial stage of the reaction, the requirement "an initial charged amount of (meth)acrylic acid is adjusted to be not more than 90 mass% of the whole use amount" can be satisfied by adjusting the amount of (meth) acrylic acid at the time that the reaction starts by raising the temperature of the reaction mixture to not more than 90% by mass of the whole use amount.

As described above, the generation of a dialkylene glycol as an impurity is inhibited in the present invention method by reducing the water content in the whole reaction mixture. In addition, as in the Examples described later, the generation amount of a dialkylene glycol can be reduced more effectively in the case that a part of the whole use amount of (meth) acrylic acid is added into a reactor at the initial stage of the reaction and then the rest (meth) acrylic acid is further added little by little, in comparison with the case of using the whole use amount all at once from the initial stage of the reaction, though the reason is not necessarily clear. It is considered to be the reason for the experimental result that the generation of a dialkylene glycol is possibly promoted by (meth) acrylic acid. Therefore, the generation of a dialkylene glycol can be inhibited more effectively by reducing the apparent amount of the acid in the reaction mixture as described above.

The order for adding a catalyst, (meth)acrylic acid and an alkylene oxide as raw material compounds is not particularly restricted, and for example, it is preferred that ethylene oxide is added to be dissolved in (meth) acrylic acid since ethylene oxide has the boiling point of 10.7°C and is in a gaseous state at room temperature. For example, it is preferred that to add an alkylene oxide into a mixture of at least a part of the whole use amount of a catalyst and (meth)acrylic acid.

The whole use amount of an alkylene oxide may be added all at once. However, it is preferred to add an alkylene oxide gradually or continuously, and it is more preferred to add an alkylene oxide continuously, since combustion may possibly occur when a large amount of unreacted alkylene oxide exists.

When a part of the whole use amount of an alkylene oxide is added into a reactor together with an initial charged amount of (meth) acrylic acid to be reacted, it is preferred to add the rest raw material (meth) acrylic acid and the rest raw material alkylene oxide not less than 0.01 hours and not more than 5 hours after the initial stage of the reaction. The period from the initial stage of the reaction is more preferably not less than 0.1 hours and not more than 5 hours. When the period is out of the above range, a dialkylene glycol mono (meth) acrylate, which is a bimolecular adduct of an alkylene oxide, may be readily formed and thereby distillation yield or purity may be possibly decreased.

With respect to the addition of (meth) acrylic acid and the rest raw material alkylene oxide, the addition time is preferably not less than 0.1 hours and not more than 5 hours, more preferably not less than 0.1 hours and not more than 4 hours, and even more preferably not less than 0.1 hours and not more than 3 hours. When the addition time is less than 0.1 hours, the amount of reaction heat generated per hour becomes large. For that reason, economic disadvantages may be possibly caused. For example, a heat exchanger for cooling is required to be enlarged. When the addition time exceeds 5 hours, productivity may be possibly lowered.

The reaction can be started by bringing the temperature of a mixture containing at least a part of the whole use amount of a catalyst, (meth) acrylic acid and an alkylene oxide up to not less than 40 °C. More specifically, the mixture may be heated to not less than 40°C, or alternatively, a mixture containing at least a part of the whole use amount of a catalyst and (meth)acrylic acid may be heated to not less than 40°C, and then an alkylene oxide is added thereto.

The reaction temperature is usually adjusted to be not less than 40°C and not more than 120°C. When the reaction temperature is less than 40°C, a gas concentration of unreacted alkylene oxide in a gas phase may become high due to an excessively low reaction rate and therefore an explosion may possibly occur. For that reason, a complicated operation may be required in order to secure safety. For example, it is required to reduce the gas concentration of an alkylene oxide in a gas phase by diluting the gas phase with an inert gas. In such a case, a designed pressure of a reactor is required to be higher, so that economic disadvantages may possibly be caused. Alternatively, a concentration of unreacted alkylene oxide may be reduced by lowering a rate of adding an alkylene oxide, but as a result, a longer reaction time may be required and therefore the productivity may be possibly lowered. On the other hand, when the reaction temperature exceeds 120°C, it may be possible to be difficult to suppress the by-production of impurities. The reaction temperature is more preferably not less than 50°C, even more preferably not less than 60°C, and even more preferably not less than 70°C, and more preferably not more than 110°C, and even more preferably not more than 100°C.

The reaction time may also be adjusted appropriately. For example, the whole use amount of raw material compounds are all supplied to a reactor and then the reaction is carried out for a period of not less than 30 minutes and not more than 10 hours. When (meth)acrylic acid and the like are gradually supplied into a reactor, after supplying the whole use amount of the compounds, the mixture of raw material compounds may be reacted for a period of not less than 30 minutes and not more than 10 hours.

A pressure within the reactor during the reaction may be adjusted depending on the kinds and the use proportions of the raw material compounds to be used, and in general the reaction is preferably carried out under an increased pressure. However, there is some danger that a combustion range is enlarged in connection with such increase of reaction pressure. Although the reaction varies depending on the initial charged amount of the raw material compounds, the initial pressure, the compression of the gas phase within the reactor caused by the addition of the raw material compounds after the initial stage of the reaction, and the partial pressure of unreacted alkylene oxide, for example, the pressure in gauge pressure is preferably not less than 0.1 MPa and not more than 1.5 MPa, and more preferably not less than 0.1 MPa and not more than 1.0 MPa.

The reaction may be stopped when an amount of (meth)acrylic acid which remains in the reaction mixture is measured and the measured amount becomes not more than the prescribed value relative to the whole reaction mixture. The prescribed amount is preferably not more than 0.2% by mass, and more preferably not more than 0.1% by mass. The reaction can be stopped by cooling the reaction mixture down to less than 60°C.

After the reaction is stopped, a hydroxyalkyl (meth)acrylate as the target compound is collected by distillation from the reaction mixture. Specifically, a distillation method using a general distillation column, a fractionating column such as a packed column, a bubble column and a perforated plate column, or the like may be employed. The above-described method is not restrictive. When distillation is carried out, other purification devices such as a rotary thin layer type evaporator may also be used in combination. The condition of distillation may be adjusted appropriately, and for example, an absolute pressure may be adjusted to not less than 1 hPa and not more than 50 hPa, temperature may be adjusted to not less than 50°C and not more than 120°C, and distillation period may be adjusted to not less than 0.5 hours and not more than 24 hours. The absolute pressure is preferably not more than 20 hPa, and more preferably not more than 10 hPa. The distillation temperature is preferably not less than 60° C and not more than 100°C. The distillation period is preferably not less than 1 hour, and preferably not more than 12 hours, more preferably not more than 6 hours, even more preferably not more than 3 hours.

Gas bubbling is carried out during distillation in order to prevent bumping by stirring the mixture. The amount of such gas bubbling is not particularly limited, and it is preferred to adjust the amount depending on various conditions since, for example, a too large amount of bubbling may become a load during distillation under a high vacuum condition. For example, the amount of gas supplied for bubbling is preferably not more than 10 vol% and not less than 0.1 volume%, relative to an evaporated volume. In regard to the gas for bubbling, it is preferred to use a gas having a polymerization prevention activity, for example, a mixed gas of an inert gas such as nitrogen with oxygen, air, and oxygen. The gas to be bubbled is preferably a dry gas from which moisture is preliminarily removed.

An attempt to distill all of the generated hydroxyalkyl (meth) acrylate may result in an increased possibility of impurity contamination. Therefore, for example, if a hydroxyalkyl methacrylate is distilled batchwise, distillation is preferably stopped when not more than 98% by mass of the generated hydroxyalkyl methacrylate is distilled. In the case of a hydroxyalkyl acrylate, distillation is preferably stopped when not more than 95% by mass of the generated hydroxyalkyl acrylate is distilled. The ratio in the case of a hydroxyalkyl methacrylate is more preferably not more than 95% by mass, and the ratio in the case of hydroxyalkyl acrylate is more preferably not more than 92% by mass. The lower limit of the ratio is not particularly limited, and is preferably not less than 80% by mass, more preferably not less than 85% by mass, and particularly preferably not less than 88% by mass, since productivity may be possibly lowered when the ratio is excessively low.

In the residue resulting from distillation of a hydroxyalkyl (meth)acrylate as the target compound, a catalyst is contained, and a polymerization inhibitor remains in some cases. In the present invention, the residue of the reaction mixture remaining after distillation is preferably used in the next and succeeding reaction as the whole or a part of a catalyst and a polymerization inhibitor. By such an embodiment, the production cost can be reduced by decreasing the use amount of a catalyst and the like, and water derived from a raw material compound can be prevented from being newly brought in.

The residue which remains after distilling off a hydroxyalkyl (meth)acrylate is usually a liquid. However, it is concerned that liquid properties of the residue may be deteriorated due to thermal history. For example, viscosity of the residue and the amount of a by-product may be increased. Therefore, the temperature of the residue is preferably maintained at not more than the distillation temperature, and when the residue is stored for a long period of time such as five days or more, the temperature is preferably maintained at not more than 50°C. The polymerization inhibitor used in the reaction works effectively also during the distillation.

### EXAMPLES

Hereinafter, the examples of the present invention are described to demonstrate the present invention more specifically, but the present invention is in no way restricted by the examples, and the examples can be appropriately modified to be carried out within a range which adapts to the contents of this specification. Such a modified example is also included in the range of the present invention.

Unless otherwise noted, "%" means "mass%".

In the present invention, "distillate ratio" means a ratio by mass of a distillate amount relative to a mass of an original reaction mixture when a certain component such as a target compound is separated by purification such as distillation.

A measurement condition of gas chromatography for measuring an amount of dialkylene glycol which was contained in a sample is shown as follows.
Measurement apparatus: Gas chromatograph GC-17A, manufactured by Shimadzu Corporation
Column: DB-1701, of which inside diameter is 0.53 mm and of which length is 30 m, manufactured by J&W SCIENTIFIC
Injection temperature: 250°C

### Example 1: Accelerated test for stability of hydroxyethyl methacrylate

Each hydroxyethyl methacrylate sample was preliminarily analyzed by the above-described gas chromatography, and then a concentration of hydroquinone monomethyl ether as a polymerization inhibitor was adjusted to be 0.005 mass%. Next, a contained amount of diethylene glycol contained in each sample was calculated on the basis of the measurement result of gas chromatography.

The above sample (10 mL) was added into a test tube of which inside diameter was 18 mm and of which length was 180 mm. The test tube was immersed in an oil bath of which temperature was 100°C in air atmosphere. The mixture was visually checked at regular intervals, and it was regarded as start time of polymerization when even a part of the mixture was solidified. The period until the start of polymerization from the start of immersion was recorded. The result is shown in Table 1. The sample of Experiment No. 1 was prepared by adding diethylene glycol to the sample obtained in Example 6.

**Table 1**

| Experiment No. | Content amount of diethylene glycol (%) | Polymerization start time (hr) |
|---|---|---|
| 1 | 0.062 | 10.3 |
| 2 (Example 4) | 0.019 | 11.2 |
| 3 (Example 3) | 0.015 | 11.4 |
| 4 (Example 6) | 0.005 | 12.6 |

If the period to polymerization start time under the above-described condition is more than 11 hours, stability of hydroxyalkyl methacrylate during storage under normal conditions is sufficient. In addition, the less a contained amount of dialkylene glycol in hydroxyalkyl methacrylate is, the longer a period to polymerization start time becomes and the higher stability of hydroxyalkyl methacrylate is.

### Example 2: Accelerated test for stability of hydroxyethyl acrylate

Each hydroxyethyl acrylate sample was preliminarily analyzed by the above-described gas chromatography, and then a concentration of hydroquinone monomethyl ether as a polymerization inhibitor was adjusted to be 0.03 mass%. Next, a contained amount of diethylene glycol contained in each sample was calculated on the basis of the measurement result of gas chromatography.

The above sample (10 mL) was added into a test tube of which inside diameter was 18 mm and of which length was 180 mm. The test tube was immersed in an oil bath of which temperature was 110°C in air atmosphere. The mixture was visually checked at regular intervals, and it was regarded as polymerization start time when even a part of the mixture was solidified. The period until the start of polymerization from the start of immersion was recorded. The result is shown in Table 2. The samples of Experiment Nos. 5 and 6 were prepared by adding diethylene glycol to the sample obtained in Example 9.

**Table 2**

| Experiment No. | Content amount of diethylene glycol (%) | Polymerization start time (hr) |
|---|---|---|
| 5 | 0.101 | 79.5 |
| 6 | 0.05 | 81.1 |
| 7 (Example 8) | 0.02 | 83.5 |
| 8 (Example 9) | 0.009 | 84.8 |

If the period to polymerization start time under the above-described condition is more than 80 hours, stability of hydroxyalkyl acrylate is sufficiently high. In particular, when a contained amount of diethylene glycol was 0.05%, stability at normal temperature was very excellent, since the period to a polymerization start time was longer by about 2 hours than the case of 0.101%.

From the above results, it was experimentally demonstrated that stability of hydroxyalkyl acrylate is sufficient, when a contained amount of dialkylene glycol is not more than 0:05 mass%.

### Example 3: Production of hydroxyethyl methacrylate

### (1) Water content in raw material compound and the like

The water contents of methacrylic acid, chromium acetate as a catalyst, and phenothiazine as a polymerization inhibitor were measured by Karl Fischer's method using a Karl Fischer's moisture meter. The water content in ethylene oxide as a raw material compound was not measured, since it is unlikely that water is mixed in ethylene oxide due to production method thereof. The water content in chromium acetate was 10.0 mass%.

### (2) Drying of raw material compound and the like

The water content of chromium acetate was the highest among the above compounds of which water contents were measured. Therefore, chromium acetate was dried at 105°C for 30 minutes using a natural convection type drying oven manufactured by Yamato Scientific Co., Ltd., and then the water content of the dried chromium acetate was measured again under the same condition described in the above (1). The water contents of each compound are shown in Table 3. When water was not detected by the above-described method, such a case was indicated as 0% in the table.

**Table 3**

| | Water content |
|---|---|
| Methacrylic acid | 0.010% |
| Ethylene oxide | 0% |
| Chromium acetate | 5.0% |
| Phenothiazine | 0% |
| Reaction mixture | 0.020% |

### (3) Reaction

The above methacrylic acid (700.0 g), the above chromium acetate (2.8 g, 0.15 mol% of the methacrylic acid) and the above phenothiazine (1.0 g) were added into an autoclave of which capacity was 2 L, which was equipped with an agitator and which was made of SUS-316. After the gas phase in the autoclave was replaced by nitrogen gas, the temperature of the mixture was increased to 80°C and the internal pressure was increased to 0.1 MPa in gauge pressure. Then, while the temperature of the mixture was maintained at 80°C, the above ethylene oxide (379.7 g, molar ratio relative to the methacrylic acid was 1.06) was added at a rate of 126.6 g/h over about 3 hours. After the addition of the ethylene oxide, the reaction was carried out for 1.5 hours while the reaction temperature was maintained at 80°C. As a result, the concentration of unreacted methacrylic acid in the whole reaction mixture became 0.10%. Then, the reaction mixture was cooled down to 40°C.

After the reaction, the reaction mixture was transferred to a glass round-bottom flask of which capacity was 2 L. The flask was set in a vacuum distillation apparatus. While air was bubbled at a rate of 10 mL/min, hydroxyethyl methacrylate was distilled under a pressure of 2 to 10 hPa at an inside temperature of 60 to 100°C for 2 hours until a distillate ratio became 95%.

The obtained hydroxyethyl methacrylate was analyzed by gas chromatography; as a result, a contained amount of diethylene glycol as an impurity was 0.012%.

### Example 4: Production of hydroxyethyl methacrylate

Hydroxyethyl methacrylate was produced in the same condition as Example 3(3) except that raw material compounds shown in Table 4 were used. The obtained hydroxyethyl methacrylate was analyzed by gas chromatography; as a result, the contained amount of diethylene glycol as an impurity was 0.019%.

**Table 4**

| | Water content |
|---|---|
| Methacrylic acid | 0.020% |
| Ethylene oxide | 0% |
| Chromium acetate | 10.0% |
| Phenothiazine | 0% |
| Reaction mixture | 0.039% |

### Example 5: Production of hydroxyethyl methacrylate

As Table 5, the same raw material compounds as those of Example 3 were used except that phenothiazine of which water content was 0.050% was used as a polymerization inhibitor. However, the water content in the whole reaction mixture was numerically the same as that in Example 3, since the use amount of phenothiazine was less than the other compounds.

**Table 5**

| | Water content |
|---|---|
| Methacrylic acid | 0.010% |
| Ethylene oxide | 0% |
| Chromium acetate | 5.0% |
| Phenothiazine | 0.050% |
| Reaction mixture | 0.020% |

The above methacrylic acid (233.3 g out of 700.0 g), the above chromium acetate (2.8 g, 0.45 mol% of the initial charged amount of the methacrylic acid) and the above phenothiazine (1.0 g) were added into an autoclave of which capacity was 2 L, which was equipped with an agitator and which was made of SUS-316. After the gas phase in the autoclave was replaced by nitrogen gas, the temperature of the mixture was increased to 80°C and the internal pressure was increased to 0.1 MPa in gauge pressure. Then, while the temperature of the mixture was maintained at 80°C, the above ethylene oxide (125.4 g out of 376.1 g, of which molar ratio relative to the whole amount of the methacrylic acid was 1.05) was added at a rate of 125.4 g/h over 1 hour. When the addition of ethylene oxide was completed, the concentration of the unreacted methacrylic acid was 4%. Then, while the temperature of the mixture was maintained at 80°C, the rest of the methacrylic acid (466.7 g) was added at a rate of 233.4 g/h over about 2 hours and ethylene oxide (250.7 g) was added at a rate of 125.4 g/h over about 2 hours. After the addition, the reaction was carried out for 2 hours while the reaction temperature was maintained at 80°C. As a result, the concentration of unreacted methacrylic acid became 0.10%. Then, the reaction mixture was cooled down to 40°C.

After the reaction, the reaction mixture was transferred to a glass round-bottom flask of which capacity was 2 L. The flask was set in a vacuum distillation apparatus. While air was bubbled at a rate of 10 mL/min, hydroxyethyl methacrylate was distilled under a pressure of 2 to 10 hPa at an inside temperature of 60 to 100°C for 2 hours until a distillate ratio became 95%.

The obtained hydroxyethyl methacrylate was analyzed by gas chromatography; as a result, a contained amount of diethylene glycol as an impurity was 0.003%. Although the reason why such a result could be obtained is not necessarily clear, it was clarified that when (meth)acrylic acid is added gradually and/or continuously, compared with the case of using the whole amount of (meth) acrylic acid from the start of the reaction, the generation of diethylene glycol can be inhibited more effectively.

### Example 6: Production of hydroxyethyl methacrylate

The dried raw material compounds described in Table 5 were used similarly to Example 5.

The above methacrylic acid (233.3 g out of 700.0 g), the above chromium acetate (2.8 g, 0.45 mol% of the initial charged amount of the methacrylic acid) and the above phenothiazine (1.0 g) were added into an autoclave of which capacity was 2 L, which was equipped with an agitator and which was made of SUS-316. After the gas phase in the autoclave was replaced by nitrogen gas, the temperature of the mixture was increased to 80°C and the internal pressure was increased to 0.1 MPa in gauge pressure. Then, while the temperature of the mixture was maintained at 80°C, the above ethylene oxide (125.4 g out of 376.1 g, of which molar ratio relative to the whole amount of the methacrylic acid was 1.05) was added at a rate of 125.4 g/h over 1 hour. Then, the temperature of the reaction mixture was raised from 80°C to 90°C over 10 minutes. While the temperature of the mixture was maintained at 90°C, the rest of the methacrylic acid (466.7 g) was added at a rate of 233.4 g/h over about 2 hours and ethylene oxide (250.7 g) was added at a rate of 125.4 g/h over about 2 hours. After the addition, the reaction was carried out for 1 hour while the reaction temperature was maintained at 90°C. As a result, the concentration of unreacted methacrylic acid became 0.10%. Then, the reaction mixture was cooled down to 40°C.

After the reaction, the reaction mixture was transferred to a glass round-bottom flask of which capacity was 2 L. The flask was set in a vacuum distillation apparatus. While air was bubbled at a rate of 10 mL/min, hydroxyethyl methacrylate was distilled under a pressure of 2 to 10 hPa at an inside temperature of 60 to 100°C for 2 hours until a distillate ratio became 95%.

The obtained hydroxyethyl methacrylate was analyzed by gas chromatography; as a result, a contained amount of diethylene glycol as an impurity was 0.005%.

### Example 7: Production of hydroxyethyl methacrylate

The raw material compounds used in Example 3 were similarly used as shown in Table 6. In Example 7, distillation residue obtained in Example 5 was reused; therefore, the water content of the distillation residue was also measured similarly to Example 3. The total water content of the compounds was numerically less than that of Example 5, since the use amount of the chromium acetate, of which water content was relatively high, was decreased in comparison with Example 5 due to the reuse of the distillation residue.

**Table 6**

| | Water content |
|---|---|
| Methacrylic acid | 0.010% |
| Ethylene oxide | 0% |
| Chromium acetate | 5.0% |
| Phenothiazine | 0% |
| Distillation residue of Example 5 | 0% |
| Reaction mixture | 0.010% |

Hydroxyethyl methacrylate was produced in the same condition as Example 5 except that the distillation residue (36 g, in which 1.9 g of chromium acetate was contained) which was obtained in Example 5 in addition to the above methacrylic acid (233.3 g out of 700.0 g), the above chromium acetate (0.9 g) and the above phenothiazine (0.3 g) were supplied into a 2 L autoclave, which was equipped with an agitator and made of SUS-316.

The obtained hydroxyethyl methacrylate was analyzed by gas chromatography; as a result, a contained amount of diethylene glycol as an impurity was 0.003%. It was demonstrated from the result that when distillation residue obtained from other reaction is reused, the water content of raw material compounds can be reduced and the generation of diethylene glycol can be inhibited more effectively.

### Example 8: Production of hydroxyethyl acrylate

The water contents of acrylic acid, chromium acetate and phenothiazine were measured by the same method as that of Example 3(1). As a result, the water content of chromium acetate was the highest among the above compounds of which water contents were measured. The chromium acetate was dried at 105°C for 30 minutes under the same condition as that of Example 3(2), and the water content of the dried chromium acetate was similarly measured again. The water contents of each compound were shown in Table 7.

**Table 7**

| | Water content |
|---|---|
| Acrylic acid | 0.050% |
| Ethylene oxide | 0% |
| Chromium acetate | 5.0% |
| Phenothiazine | 0% |
| Reaction mixture | 0.046% |

The above acrylic acid (233.3 g out of 700.0 g), the above chromium acetate (3.5 g, 0.47 mol% of the initial charged amount of the acrylic acid) and the above phenothiazine (1.0 g) were added into an autoclave of which capacity was 2 L, which was equipped with an agitator and which was made of SUS-316. After the gas phase in the autoclave was replaced by nitrogen gas, the temperature of the mixture was increased to 80°C and the internal pressure was increased to 0.1 MPa in gauge pressure. Then, while the temperature of the mixture was maintained at 80°C, the above ethylene oxide (157.2 g out of 449.3 g, of which molar ratio relative to the whole acrylic acid was 1.05) was added at a rate of 224.6 g/h over about 0.7 hours. While the reaction temperature was maintained at 80°C, the rest of the acrylic acid (466.7 g) was added at a rate of 359 g/h over about 1.3 hours and ethylene oxide (292.1 g) was added at a rate of 224.6 g/h over about 1.3 hours. After the addition, the reaction was carried out for 3 hours while the temperature was maintained at 80°C. As a result, the concentration of unreacted acrylic acid became 0.05%. Then, the reaction mixture was cooled down to 40°C.

After the reaction, the reaction mixture was transferred to a glass round-bottom flask of which capacity was 2 L. The flask was set in a vacuum distillation apparatus. While air was bubbled at a rate of 10mL/min, hydroxyethyl acrylate was distilled under a pressure of 2 to 10 hPa at an inside temperature of 60 to 100°C for 2 hours until a distillate ratio became 90%.

The obtained hydroxyethyl acrylate was analyzed by gas chromatography; as a result, a contained amount of diethylene glycol as an impurity was 0.02%.

### Example 9: Production of hydroxyethyl acrylate

The dried raw material compounds used in Example 8 were used as shown in Table 8. In Example 9, the distillation residue obtained in Example 8 was reused; therefore, the water content of the distillation residue was also measured similarly to Example 3. The total water content of the compounds was numerically less than that of Example 8, since the use amount of the chromium acetate, of which water content was relatively high, was decreased in comparison with Example 8 due to the reuse of the distillation residue.

**Table 8**

| | Water content |
|---|---|
| Acrylic acid | 0.050% |
| Ethylene oxide | 0% |
| Chromium acetate | 5.0% |
| Phenothiazine | 0% |
| Distillation residue of Example 8 | 0% |
| Reaction mixture | 0.029% |

The distillation residue (103.8 g, which contained 3.1 g of chromium acetate) obtained in Example 8 in addition to the above acrylic acid (233.3 g out of 700.0 g), the above chromium acetate (0.4 g) and the above phenothiazine (0.1 g) were supplied into a 2L autoclave which was equipped with an agitator and made of SUS-316. Hydroxyethyl acrylate was produced similarly to Example 8 other than the above condition.

The obtained hydroxyethyl acrylate was analyzed by gas chromatography; as a result, a contained amount of diethylene glycol as an impurity was 0.009%. It was demonstrated from the result that when distillation residue obtained from other reaction is reused, the water content of raw material compounds can be reduced and the generation of diethylene glycol can be inhibited more effectively.

### Comparative Example 1: Production of hydroxyethyl acrylate

The raw material compounds shown in Table 9 were used to produce hydroxyethyl acrylate.

**Table 9**

| | Water content |
|---|---|
| Acrylic acid | 0.10% |
| Ethylene oxide | 0% |
| Chromium acetate | 5.0% |
| Phenothiazine | 0% |
| Reaction mixture | 0.081% |

The above acrylic acid (572.0 g), the above chromium acetate (3.8 g, 0.21 mol% of the acrylic acid) and the above phenothiazine (0.5 g) were added into an autoclave of which capacity was 2 L, which was equipped with an agitator and which was made of SUS-316. After the gas phase in the autoclave was replaced by nitrogen gas, the temperature of the mixture was increased to 50°C and the internal pressure was increased to 0.1 MPa in gauge pressure. Then, while the temperature of the mixture was maintained at 50°C, the above ethylene oxide (367.0 g, molar ratio relative to the acrylic acid was 1.05) was added at a rate of 91. 8 g/h over about 4 hours. After the addition of the ethylene oxide, the reaction temperature was increased to 70°C. The reaction was carried out for 3 hours while the reaction temperature was maintained at 70°C. As a result, the concentration of unreacted acrylic acid in the whole reaction mixture became 0.10%. Then, the reaction mixture was cooled down to 40°C.

After the reaction, the reaction mixture was transferred to a glass round-bottom flask of which capacity was 2 L. The flask was set in a vacuum distillation apparatus. While air was bubbled at a rate of 10 mL/min, hydroxyethyl acrylate was distilled under a pressure of 2 to 10 hPa at an inside temperature of 60 to 80°C for 2 hours until a distillate ratio became 87%.

The obtained hydroxyethyl acrylate was analyzed by gas chromatography; as a result, a contained amount of diethylene glycol as an impurity was 0.071%. It was clear from the result that when the water content in the whole reaction mixture exceeds 0.05 mass%, the generation amount of diethylene glycol as an impurity is increased.

## Claims

1. A method for producing a hydroxyalkyl (meth)acrylate, comprising the step of reacting (meth)acrylic acid with an alkylene oxide in the presence of a catalyst, wherein a water content in a whole reaction mixture is adjusted to be not more than 0.05 mass%.

2. The method according to claim 1, an initial charged amount of the (meth)acrylic acid is adjusted to be not more than 90 mass% of the whole use amount, and after all of or a part of the alkylene oxide is added, the rest (meth)acrylic acid is added.

3. The method according to claim 1 or 2, after the (meth)acrylic acid is reacted with the alkylene oxide, the hydroxyalkyl (meth)acrylate is separated as a target compound from the reaction mixture, and then the residual reaction mixture is used in the next reaction.

## Patentansprüche

1. Verfahren zur Herstellung eines Hydroxyalkyl(meth)acrylats, umfassend den Schritt des Umsetzens von (Meth)acrylsäure mit einem Alkylenoxid in der Gegenwart eines Katalysators,
wobei ein Wassergehalt in einem gesamten Reaktionsgemisch so eingestellt ist, dass er nicht mehr als 0,05 Massen-% beträgt.

2. Verfahren nach Anspruch 1, wobei eine anfänglich dosierte Menge der (Meth)acrylsäure so eingestellt ist, dass sie nicht mehr als 90 Massen-% von der insgesamt verwendeten Menge beträgt, und, nachdem das gesamte oder ein Teil des Alkylenoxids hinzugefügt wurde, der Rest (Meth)acrylsäure hinzugefügt wird.

3. Verfahren nach Anspruch 1 oder 2, wobei, nachdem die (Meth)acrylsäure mit dem Alkylenoxid umgesetzt wurde, das Hydroxyalkyl(meth)acrylat als eine Zielverbindung von dem Reaktionsgemisch abgetrennt wird und anschließend das restliche Reaktionsgemisch in der nächsten Umsetzung verwendet wird.

## Revendications

1. Méthode de production d'un (méth)acrylate d'hydroxyalkyle, comprenant l'étape de réaction d'acide (méth)acrylique avec un oxyde d'alkylène en présence d'un catalyseur, où la teneur en eau dans un mélange réactionnel total est ajustée pour ne pas être supérieure à 0,05% en masse.

2. Méthode selon la revendication 1, où une quantité initiale chargée en acide (méth)acrylique est ajustée pour ne pas être supérieure à 90% en masse de la quantité totale utilisée, et après que tout ou partie de l'oxyde d'alkylène est ajouté, le reste d'acide (méth)acrylique est ajouté.

3. Méthode selon la revendication 1 ou 2, où après que l'acide (méth)acrylique soit réagi avec l'oxyde d'alkylène, le (méth)acrylate d'hydroxyalkyle est séparé du mélange réactionnel en tant que composé cible, et ensuite le mélange réactionnel résiduel est utilisé dans la réaction suivante.
